# EUROPEAN PATENT SPECIFICATION

(11) **EP 3 789 029 B1**
(45) Date of publication and mention of the grant of the patent: **08.05.2024**
(21) Application number: 19796396.0
(22) Date of filing: 23.04.2019
(51) Int. Cl.: A61K 31/546, A61K 31/4515, A61K 31/496, A61P 43/00, A23L 33/10, A61K 31/5415, A61P 35/00, A61K 45/06

(54) **RADIATION SENSITIVITY ENHANCING COMPOSITION CONTAINING ARIPIPRAZOLE AS ACTIVE INGREDIENT**
ZUSAMMENSETZUNG ZUR ERHÖHUNG DER STRAHLUNGSEMPFINDLICHKEIT MIT ARIPIPRAZOL ALS WIRKSTOFF
COMPOSITION AMÉLIORANT LA SENSIBILITÉ AU RAYONNEMENT CONTENANT DE L'ARIPIPRAZOLE COMME PRINCIPE ACTIF

(30) Priority: 04.05.2018 KR 20180051702; 13.03.2019 KR 20190028720
(43) Date of publication of application: 10.03.2021
(60) Divisional application: 22176241.2
(73) Proprietor: Korea Institute of Radiological & Medical Sciences, Seoul 01812 (KR)
(72) Inventor: LIM, Young Bin, Seoul 01820 (KR); PARK, Myung Jin, Seoul 02054 (KR); LEE, Hyoun Ji, Seoul 06798 (KR)
(74) Representative: Roos, Peter
(86) International application number: PCT/KR2019/004900
(87) International publication number: WO 2019/212185

(56) References cited:
- KR-A- 20090 065 514
- KR-A- 20170 026 115
- US-A1- 2007 099 905
- LI, H. et al.: "Thioridazine sensitizes esophageal carcinoma cell lines to radiotherapy-induced apoptosis in vitro and in vivo", Medical Science Monitor., vol. 22, 2016, pages 2624-2634, XP055648597,
- SUZUKI, S. et al.: "Aripiprazole, an antipsychotic and partial dopamine agonist, inhibits cancer stem cells and reverses chemoresistance", Anticancer Research, vol. 36, no. 10, 2016, pages 5153-5161, XP055405053,

## Description

### [Technical Field]

The present invention relates to a composition for enhancing radiation sensitivity containing aripiprazole as an active ingredient, and more specifically, to a composition for enhancing radiation sensitivity capable of treating cancer by acting as a radiation sensitizer when aripiprazole is combined with radiation.

### [Background Art]

Cancer therapies can be broadly categorized into surgery, radiation therapy, and chemotherapy. As the number of cancer patients receiving radiation therapy worldwide is increasing every year and thus the importance of radiation therapy in cancer treatment is also increasing, but the therapy is accompanied by side effects that reduce the efficiency of treatment, such as acquisition of radiation resistance of cancer cells and damage to normal tissues during high-dose radiation treatment. In order to reduce the side effects of radiation and overcome the resistance thereto, research on radiation sensitizers that increase the anticancer effect of radiation when administered in combination with radiation are being developed.

Aripiprazole has the chemical name 7-{4-[4-(2,3-dichlorophenyl)-1-piperazinyl]-butoxy}-3,4-dihydro carbostyryl or 7-{4-[4-(2,3-dichlorophenyl)-1-piperazinyl]-butoxy}-3,4-dihydro-2(1H)-quinolinone. Aripiprazole is a strong partial agonist of the dopamine D2 receptor, and is known to be useful in the treatment of schizophrenia, bipolar disorder and clinical depression as an antipsychotic drug. Until now, the pharmacological action of aripiprazole has been limitedly studied on the central nervous system and neurological diseases, and its technology has also been researched with focusing only on increasing the solubility and absorption rate of the drug, and the radiation-sensitizing effect of aripiprazole is not known at all and no research on this was done. Therefore, there is an urgent need for research and development on the radiation sensitizing effect of aripiprazole. KR 2017 0026 115 A relates to a composition for preventing or treating cancer comprising aripiprazole as an active ingredient. LI, H. et al., "Thioridazine sensitizes esophageal carcinoma cell lines to radiotherapy-induced apoptosis in vitro and in vivo", Medical Science Monitor., vol. 22, pages 2624 - 2634 relates to a study about a combined treatment of ESCC cells with thioridazine and irradiation. SUZUKI, S. et al., "Aripiprazole, an antipsychotic and partial dopamine agonist, inhibits cancer stem cells and reverses chemoresistance", Anticancer Research, vol. 36, no. 10, pages 5153 - 5161 relates to a study about aripiprazole as an anticancer stem cell drug. US 2007/099905 A1 relates to a combination of drugs for the treatment of neoplasms. KR 2009 0065514 A relates to an orally deliverable pharmaceutical composition for the controlled release of aripiprazole.

### [Disclosure]

### [Technical Problem]

An object of the present invention is to provide a composition for use in enhancing radiation sensitivity comprising aripiprazole or a pharmaceutically acceptable salt thereof as an active ingredient.

Also, an object of the present invention is to provide a composition for use in enhancing radiation sensitivity comprising: aripiprazole or a pharmaceutically acceptable salt thereof and a dopamine receptor inhibitor, as an active ingredient.

### [Technical Solution]

In order to achieve the above object, the present invention provides a composition according to claim 1.

### [Advantageous Effects]

The present invention relates to a composition for use in enhancing radiation sensitivity containing aripiprazole as an active ingredient, and more specifically, to a composition for use in enhancing radiation sensitivity capable of treating cancer by acting as a radiation sensitizer when aripiprazole is combined with radiation. By administering an effective amount of aripiprazole according to the present invention in combination with radiation irradiation, it has excellent radiation sensitivity enhancing effects such as the reduction of cancer cell viability and the induction of cancer cell death, and so it can be usefully used as a radiation sensitivity enhancer.

### [Description of Drawings]

FIG. 1 shows the effect of treatment by 321 drugs alone or in combination with radiation in MCF-7 cells on cell viability.
FIG. 2 shows the effect of treatment with various concentrations of aripiprazole alone or in combination with radiation in MCF-7 cells on cell viability.
FIG. 3 shows the effect of aripiprazole alone or in combination with radiation treatment in MCF-7 cells on PARP cleavage.
FIG. 4 shows the effect of aripiprazole alone or in combination with radiation treatment in MCF-7 or U251 cells on DNA fragmentation.
FIG. 5 shows the results of evaluating PARP cleavage after treatment of thioridazine or aripiprazole in combination with radiation in MCF-7 cells, respectively, or treatment of thioridazine and aripiprazole at the same time in combination with radiation.
FIG. 6 shows the results of evaluating DNA fragmentation after treatment of thioridazine or aripiprazole in combination with radiation in MCF-7 or BT-474 cells, respectively, or treatment of thioridazine and aripiprazole at the same time in combination with radiation.
FIG. 7 shows the results of evaluating PARP cleavage after treatment of haloperidol or aripiprazole in combination with radiation, respectively, or haloperidol and aripiprazole at the same time in combination with radiation in MCF-7 cells.
FIG. 8 shows the results of evaluating DNA fragmentation after treatment of haloperidol or aripiprazole in combination with radiation, respectively, or haloperidol and aripiprazole at the same time in combination with radiation in MCF-7 or BT-474 cells.

### [Best Mode]

The present invention provides a composition for use in enhancing radiation sensitivity comprising aripiprazole or a pharmaceutically acceptable salt thereof as an active ingredient.

The composition for use in enhancing radiation sensitivity may further comprise a dopamine receptor inhibitor, as an active ingredient. Preferably, the dopamine receptor inhibitor may be thioridazine or haloperidol, but it is not limited thereto.

Preferably, the composition can induce apoptosis of cancer cells by irradiation, and more preferably, the composition can induce PARP cleavage or DNA fragmentation of cancer cells.

Preferably, the composition may be administered in combination with irradiation during cancer treatment. More preferably, the cancer may be breast cancer, lung cancer, bone cancer, pancreatic cancer, skin cancer, oral cancer, oropharyngeal cancer, uterine cancer, ovarian cancer, rectal cancer, gastric cancer, endometrial carcinoma, cervical carcinoma, vaginal carcinoma, small intestine cancer, thyroid cancer, parathyroid cancer, prostate cancer, chronic or acute leukemia, lymphocyte lymphoma, bladder cancer, kidney cancer, liver cancer, colon cancer or brain tumors, but it is not limited thereto.

In the present invention, the term "pharmaceutically acceptable salt" refers to a salt having a safety and efficacy profile suitable for administration to humans. Specifically, the specific forms of a pharmaceutically acceptable salt of aripiprazole include salts derived from inorganic acids such as hydrochloric acid, nitric acid, phosphoric acid, sulfuric acid, hydrobromic acid, hydroiodic acid, phosphorous acid and mixtures thereof, as well as salts derived from organic acids such as aliphatic mono- and dicarboxylic acids, phenyl-substituted alkanoic acids, hydroxyl alkanoic acids, alkanedioic acids, aromatic acids, and aliphatic and aromatic sulfonic acids, but they are not limited thereto.

In the present invention, the term "radiation sensitivity" refers to the number of surviving cells based on radiation dose. In the present invention, in order to measure radiation sensitivity, it was confirmed by using a method of measuring the number of colonies formed after irradiating radiation by dose.

In the present invention, the term "enhancement" refers to a decrease in the number of cells surviving at a certain radiation dose, a decrease in radiation dose required for a lethal dose, or a combination thereof, but also includes other conventional means of enhancement are also included. Specifically, in the present invention, the increase in sensitivity of the radiation means that when the number of colonies formed after radiation by dose is measured, the number of colonies decreases according to the radiation concentration, or the value of the slope of the created linear model increases.

In the present invention, the term "irradiation" refers to a tropical treatment method that damages DNA of malignant cells. Normal cells have a greater ability to repair this damage than tumor cells. Irradiation refers to a treatment using these differences, and it includes a method of treatment using radiation in a conventional sense. Any references in the description to methods of treatment refer to the compounds and compositions of the present invention for use in a method for treatment of the human (or animal) body by therapy,

Since irradiation is a form of topical therapy, side effects are generally limited to the treated area. However, there is fatigue as a common systemic symptom. While it is true that many genetic factors predispose to secondary cancer in some patients, radiation also contributes to the increased risk involved. The composition for increasing radiation sensitivity according to the present invention can reduce such side effects by reducing the required amount of radiation. In addition, radiation sensitivity can be increased not only in radiation-sensitive cancer cells, but also in radiation-resistant cancer cells.

In the present invention, the term "administration in combination" means administering together with radiation irradiation in an anticancer therapy for treating various types of cancer cells. Specifically, radiation irradiation treatment may be combined in an anticancer therapy for treating cancer cells such as solid cancer such as lung cancer, breast cancer, colon cancer, ovarian cancer, head and neck cancer or brain cancer.

When the composition of the present invention is a pharmaceutical composition, the pharmaceutical composition may include a pharmaceutically acceptable carrier other than aripiprazole, and such a pharmaceutically acceptable carrier is commonly used in pharmaceutical formulations and include lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, mineral oil, and the like, but it is not limited thereto. In addition, the pharmaceutical composition may further include lubricants, wetting agents, sweetening agents, flavoring agents, emulsifying agents, suspending agents, preservatives, and the like as additives.

The method of administration of the pharmaceutical composition is determined according to the degree of symptoms, and a topical administration method is usually preferred. In addition, the dosage of the active ingredient in the pharmaceutical composition may vary depending on the route of administration, the severity of the disease, the age, the sex, and weight of the patient, and may be administered once to several times a day.

The pharmaceutical composition may be administered to mammals such as rats, mice, livestock, and humans by various routes. Any mode of administration can be expected, for example, oral, rectal or intravenous, intramuscular, subcutaneous, intrauterine dura mater or intracerebroventricular injection.

The pharmaceutical composition may be prepared in unit dosage form by formulation using a pharmaceutically acceptable carrier and/or excipient, or may be prepared by incorporating it into a multi-dose container. In this case, the formulation may be in the form of solution, suspension or emulsion, or may be in the form of elixir, extract, powder, granule, tablet, plaster, lotion, ointment and the like.

In addition, when the composition is a health functional food composition, the health functional food composition may be provided in the form of powder, granule, tablet, capsule, syrup, beverage or pill, and the health functional food composition is used together with other foods or food additives other than aripiprazole according to the present invention, which is an active ingredient, and may be appropriately used according to a conventional method. The mixing amount of the active ingredient may be appropriately determined according to the purpose of use, for example, prevention, health or therapeutic treatment.

The effective dose of aripiprazole contained in the health functional food composition may be used in accordance with the effective dose of the pharmaceutical composition, but in the case of long-term intake for the purpose of health and hygiene or health control, it may be less than the above range and it is clear that the active ingredient can be used in an amount beyond the above range because there is no problem in terms of safety.

There are no specific restrictions on the types of health functional foods, for example meat, sausage, bread, chocolate, candy, snacks, confectionery, pizza, ramen, other noodles, gum, dairy products including ice cream, various soups, beverages, tea, drinks, alcoholic beverages, and vitamin complexes, etc.

Hereinafter, the present invention will be described in more detail through examples.

### <Example 1> Detection of radiation sensitive agents in breast cancer cells

Cytotoxicity was not observed in single treatment within radiation-resistant breast cancer cells, but candidate drugs for radiation sensitizers that enhance radiation sensitivity in combination treatment with radiation were searched in the drug library of Enzo.

MCF-7 cells, a human breast cancer cell line, were adjusted to a concentration of 5000 cells/well, then inoculated into a 96 well plate and pre-cultured for 24 hours. Subsequently, the medium was removed, and each drug in Enzo's drug library at a concentration of 5 mM was treated alone or in combination with radiation. After 96 hours, the survival rate of cells was measured by Dogen's EZ-Cytox Cell Viability Assay kit.

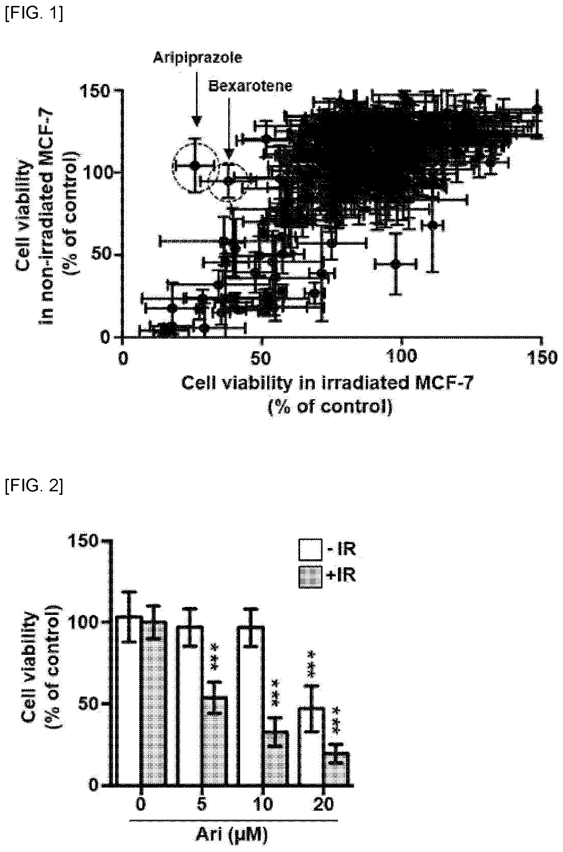

As shown in FIG. 1, aripiprazole was determined as a radiation sensitizer that lowers the survival rate of radiation-resistant cells when treated in combination with radiation, although there is no effect on the survival rate of cells in drug treatment alone.

### <Example 2> Effect of aripiprazole on radiation sensitivity of cancer cells

In order to confirm the effect of aripiprazole on radiation sensitivity in cancer cells, a cell viability measurement experiment was performed in breast cancer cells having radiation resistance using the MTT assay as follows.

After the MCF-7 cells were adjusted to a concentration of 5000 cells/well, they were inoculated into a 96-well plate and pre-cultured for 24 hours. Thereafter, the medium was removed and aripiprazole (5, 10, 20 µM) was treated alone or in combination with radiation. After 96 hours, the survival rate of cells was measured by Dogen's EZ-Cytox Cell Viability Assay kit.

As shown in FIG. 2, it was found that the survival rate reduction effect was more excellent when treated in combination with radiation than the treatment with aripiprazole alone. Aripiprazole was found to have an excellent radiation sensitizing effect in a concentration-dependent manner.

### <Example 3> Effect of aripiprazole on radiation-induced apoptosis in cancer cells

It was recently reported that aripiprazole can induce apoptosis in cancer cells and to confirm the effect of aripiprazole on radiation-induced apoptosis in cancer cells, PARP cleavage and DNA fragmentation were measured in cancer cells as follows.

The MCF-7 cells were treated with aripiprazole (1, 5, 10, 20 µM) alone or in combination with radiation. After 24 hours, after washing with PBS, proteins were extracted from the cells, and the degree of PARP cleavage was confirmed by Western blot.

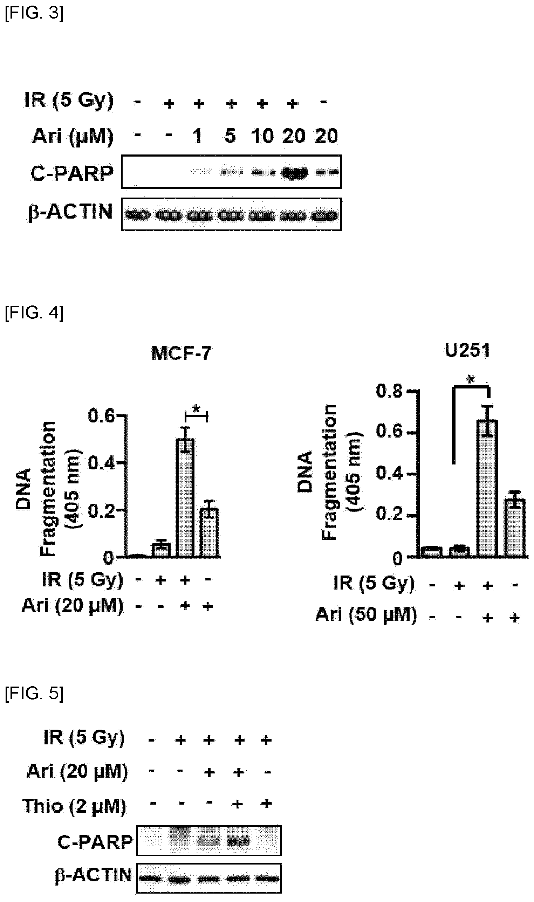

As shown in FIG. 3, it was found that the PARP cleavage induction effect was more excellent when treated in combination with radiation than treated aripiprazole alone. Aripiprazole was found to induce apoptosis in radiation-treated cancer cells in a concentration-dependent manner.

After the MCF-7 cells or U251 cells were adjusted to a concentration of 5000 cells/well, they were inoculated into a 96 well plate and pre-cultured for 24 hours. Thereafter, the medium was removed and aripiprazole was treated alone or in combination with radiation. After 24 hours, the degree of DNA fragmentation was confirmed using Roche's Cell Death Detection ELISA plus kit.

As shown in FIG. 4, it was found that when aripiprazole is treated in combination with the radiation, the DNA fragmentation inducing effect was superior to the effect of adding up each treatment. It was found that the effect of inducing apoptosis was more excellent when the two treatments were used simultaneously than the effect of inducing DNA fragmentation by single treatment.

### <Example 4> Effect of dopamine receptor inhibitor of thioridazine and aripiprazole on radiation-induced apoptosis in cancer cells

To confirm the effect of dopamine receptor inhibitors and aripiprazole on radiation-induced apoptosis in cancer cells, thioridazine or aripiprazole was treated in combination with radiation, respectively, or thioridazine and aripiprazole were treated in combination with radiation in MCF-7 cells. After 24 hours, after washing with PBS, proteins were extracted from the cells, and the degree of PARP cleavage was confirmed by Western blot.

As shown in FIG. 5, compared to the effect of inducing PARP cleavage by the treatments of 'combined treatment with thioridazine and radiation' or 'combined treatment with aripiprazole and radiation', the combined treatment of thioridazine and aripiprazole with radiation was found to exhibit more excellent PARP cleavage induction effect.

After the MCF-7 cells or BT-474 cells were adjusted to a concentration of 5000 cells/well, they were inoculated into a 96 well plate and pre-cultured for 24 hours. Thereafter, the medium was removed, and thioridazine or aripiprazole was treated in combination with radiation, respectively, or thioridazine and aripiprazole were simultaneously treated with radiation. After 24 hours, the degree of DNA fragmentation was confirmed using Roche's Cell Death Detection ELISA plus kit.

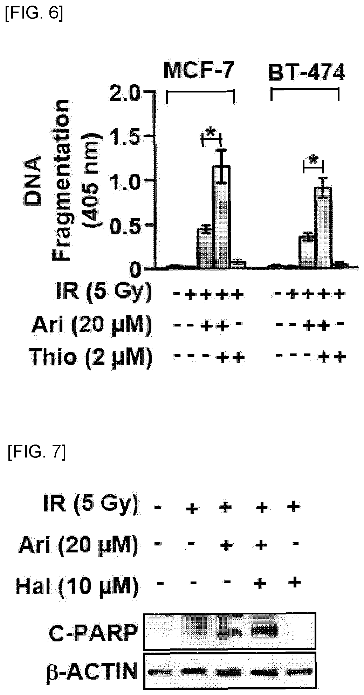

As shown in FIG. 6, the combined treatment of thioridazine and aripiprazole with radiation was found to showed superior DNA fragmentation induction effect than the effect of adding up each treatment of thioridazine in combination with radiation or treatment of aripiprazole in combination with radiation.

### <Example 5> Effect of dopamine receptor inhibitor of haloperidol and aripiprazole on radiation-induced apoptosis in cancer cells

To confirm the effect of dopamine receptor inhibitors and aripiprazole on radiation-induced apoptosis in cancer cells, haloperidol or aripiprazole was treated in combination with radiation, respectively, or haloperidol and aripiprazole were treated in combination with radiation in MCF-7 cells. After 24 hours, after washing with PBS, proteins were extracted from the cells, and the degree of PARP cleavage was confirmed by Western blot.

As shown in FIG. 7, compared to the effect of inducing PARP cleavage by the treatments of 'combined treatment with haloperidol and radiation' or 'combined treatment with aripiprazole and radiation', the combined treatment of haloperidol and aripiprazole with radiation was found to exhibit more excellent PARP cleavage induction effect.

After the MCF-7 cells or BT-474 cells were adjusted to a concentration of 5000 cells/well, they were inoculated into a 96 well plate and pre-cultured for 24 hours. Thereafter, the medium was removed, and haloperidol or aripiprazole was treated in combination with radiation, respectively, or haloperidol and aripiprazole were simultaneously treated with radiation. After 24 hours, the degree of DNA fragmentation was confirmed using Roche's Cell Death Detection ELISA plus kit.

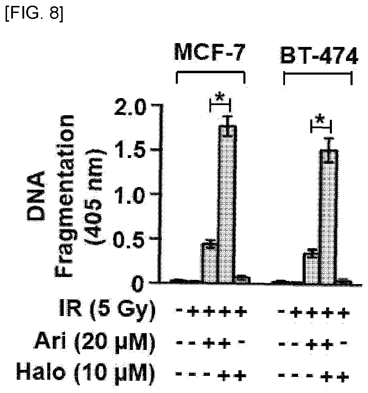

As shown in FIG. 8, the combined treatment of haloperidol and aripiprazole with radiation was found to showed superior DNA fragmentation induction effect than the effect of adding up each treatment of haloperidol in combination with radiation or treatment of aripiprazole in combination with radiation.

## Claims

1. A composition for use in enhancing radiation sensitivity comprising aripiprazole or a pharmaceutically acceptable salt thereof as an active ingredient.

2. The composition for use in enhancing radiation sensitivity of claim 1, further comprising a dopamine receptor inhibitor as an active ingredient.

3. The composition for use in enhancing radiation sensitivity of claim 2, wherein the dopamine receptor inhibitor is thioridazine or haloperidol.

4. The composition for use in enhancing radiation sensitivity of any one of claims 1 to 3, wherein the composition induces apoptosis of cancer cells by irradiation with radiation.

5. The composition for use in enhancing radiation sensitivity of claim 4, wherein the composition induces PARP cleavage or DNA fragmentation of cancer cells.

6. The composition for use in enhancing radiation sensitivity of any one of claims 1 to 3, wherein the composition is administered in combination with radiation irradiation during cancer treatment.

7. The composition for use in enhancing radiation sensitivity of claim 6, wherein the cancer is breast cancer, lung cancer, bone cancer, pancreatic cancer, skin cancer, oral cancer, oropharyngeal cancer, uterine cancer, ovarian cancer, rectal cancer, gastric cancer, endometrial carcinoma, cervical carcinoma, vaginal carcinoma, small intestine cancer, thyroid cancer, parathyroid cancer, prostate cancer, chronic or acute leukemia, lymphocyte lymphoma, bladder cancer, kidney cancer, liver cancer, colon cancer or brain tumors.

## Patentansprüche

1. Zusammensetzung zur Verwendung bei der Steigerung der Strahlungssensitivität, umfassend Aripiprazol oder ein pharmazeutisch akzeptables Salz davon als Wirkstoff.

2. Zusammensetzung zur Verwendung bei der Steigerung der Strahlungssensitivität nach Anspruch 1, ferner umfassend einen Dopaminrezeptor-Inhibitor als Wirkstoff.

3. Zusammensetzung zur Verwendung bei der Steigerung der Strahlungssensitivität nach Anspruch 2, wobei der Dopaminrezeptor-Inhibitor Thioridazin oder Haloperidol ist.

4. Zusammensetzung zur Verwendung bei der Steigerung der Strahlungssensitivität nach einem der Ansprüche 1 bis 3, wobei die Zusammensetzung die Apoptose von Krebszellen durch Bestrahlung mit Strahlung induziert.

5. Zusammensetzung zur Verwendung bei der Steigerung der Strahlungssensitivität nach Anspruch 4, wobei die Zusammensetzung die PARP-Spaltung oder DNA-Fragmentierung von Krebszellen induziert.

6. Zusammensetzung zur Verwendung bei der Steigerung der Strahlungssensitivität nach einem der Ansprüche 1 bis 3, wobei die Zusammensetzung bei einer Krebsbehandlung in Kombination mit Strahlenbestrahlung angewendet wird.

7. Zusammensetzung zur Verwendung bei der Steigerung der Strahlensensitivität nach Anspruch 6, wobei der Krebs Brustkrebs, Lungenkrebs, Knochenkrebs, Bauchspeicheldrüsenkrebs, Hautkrebs, Mundkrebs, Oropharynxkrebs, Gebärmutterkrebs, Eierstockkrebs, Rektum krebs, Magenkrebs, Endometriumkarzinom, Gebärmutterhalskrebs, Vaginalkarzinom, Dünndarmkrebs, Schilddrüsenkrebs, Nebenschilddrüsenkrebs, Prostatakrebs, chronische oder akute Leukämie, Lymphozyten-Lymphom, Blasenkrebs, Nierenkrebs, Leberkrebs, Dickdarmkrebs oder Gehirntumoren ist.

## Revendications

1. Composition pour utilisation dans l'amélioration de la sensibilité au rayonnement comprenant l'aripiprazole ou un sel pharmaceutiquement acceptable de celui-ci en tant qu'ingrédient actif.

2. Composition pour utilisation dans l'amélioration de la sensibilité au rayonnement selon la revendication 1, comprenant en outre un inhibiteur du récepteur de la dopamine en tant qu'ingrédient actif.

3. Composition pour utilisation dans l'amélioration de la sensibilité au radiations selon la revendication 2, dans laquelle l'inhibiteur du récepteur de la dopamine est la thioridazine ou l'halopéridol.

4. Composition pour utilisation dans l'amélioration de la sensibilité au rayonnement selon une des revendications 1 à 3, dans laquelle la composition induit l'apoptose des cellules cancéreuses par irradiation avec un rayonnement.

5. Composition pour utilisation dans l'amélioration de la sensibilité au rayonnements selon la revendication 4, dans laquelle la composition induit le clivage PARP ou la fragmentation de l'ADN des cellules cancéreuses.

6. Composition pour utilisation dans l'amélioration de la sensibilité au rayonnement selon une des revendications 1 à 3, dans laquelle la composition est administrée en combinaison avec l'irradiation pendant un traitement du cancer.

7. Composition pour utilisation dans l'amélioration de la sensibilité au rayonnement selon la revendication 6, dans laquelle le cancer est cancer du sein, cancer du poumon, cancer des os, cancer du pancréas, cancer de la peau, cancer de la bouche, cancer oropharyngé, cancer de l'utérus, cancer de l'ovaire, cancer du rectum, cancer de l'estomac, cancer de l'endomètre, cancer du col de l'utérus, cancer du vagin, cancer de l'intestin grêle, cancer de la thyroïde, cancer de la parathyroïde, cancer de la prostate, leucémie chronique ou aiguë, lymphome lymphocytaire, cancer de la vessie, cancer du rein, cancer du foie, cancer du côlon ou tumeurs cérébrales.
